(19) Europäisches Patentamt
European Patent Office
Office européen des brevets

(11) **EP 0 965 648 A1**

(12) **EUROPEAN PATENT APPLICATION**

(43) Date of publication:
22.12.1999 Bulletin 1999/51

(51) Int. Cl.$^6$: **C12Q 1/68**, C12N 15/12

(21) Application number: 98870125.6

(22) Date of filing: 02.06.1998

(84) Designated Contracting States:
**AT BE CH CY DE DK ES FI FR GB GR IE IT LI LU MC NL PT SE**
Designated Extension States:
**AL LT LV MK RO SI**

(71) Applicant:
**RIJKSUNIVERSITEIT LEIDEN**
**2300 RA Leiden (NL)**

(72) Inventor:
**The designation of the inventor has not yet been filed**

(74) Representative:
**De Clercq, Ann G. Y.**
**Innogenetics N.V.,**
**Industriepark Zwijnaarde 7,**
**P.O. Box 4**
**9052 Gent (BE)**

Remarks:
• The applicant has subsequently filed a sequence listing and declared, that it includes no new matter.
• Claims 11-15 are deemed to be abandoned due to non-payment of the claims fees (Rule 31 (2) EPC).

(54) **Method for genomic typing of the Minor Histocomptability Antigen HA-1.**

(57) The present invention provides a method for genomic typing of alleles of the Minor Histocompatibility Antigen HA-1 in a sample, with said method comprising: a) contacting the genomic polynucleic acids in the sample with at least one pair of primers, whereby the 5'- and/or the 3'-primer of said at least one pair of primers specifically hybridize to target regions comprising polymorphic nucleotides in said alleles, and performing an amplification reaction; b) for each of said at least one pair of primers detecting whether or not in step a) an amplification product is formed; c) inferring from the result of step b) which HA-1 allele is present in said sample. The present invention also provides a method for genomic typing of alleles of the Minor Histocompatibility Antigen HA-1 in a sample, with said method comprising: a) amplifying a fragment of said alleles, with said fragment comprising at least one polymorphic nucleotide, by use of at least one pair of primers specifically hybridizing to conserved target regions in said alleles; b) hybridizing the amplified product of step a) to at least one probe specifically hybridizing to a target region comprising one or more polymorphic nucleotides in said allele; c) inferring from the result of step b) wich HA-1 allele is present in said sample. In addition, the present invention provides primers and probes for use in the above-mentioned methods. Diagnostic kits enabling said methods are also provided.

**Fig.1 :Sequences encoding the HA-1 antigen locus**

A

HA-1 R-allele:
GTG TTG CGT GAC GAC CTC CTT GAG GCC
V   L   R   D   D   L   L   E   A

HA-1 H-allele:
GTG CTG CAT GAC GAC CTC CTT GAG GCC
V   L   H   D   D   L   L   E   A

B   exon a              intron a              exon b
GTG TTG CGT GAC G gtgagagcca//ctcactccgactctccccagcag AC CTC CTT GAG GCC

GTG TTG CGT GAC GAC CTC CTT GAG GCC
V   L   R   D   D   L   L   E   A

**Description**

BACKGROUND OF THE INVENTION

Bone Marrow Transplantation (BMT) is the present treatment for hematological malignancies. One of the major drawbacks of allogeneic BMT is Graft versus Host Disease (GvHD). In recipients of allogeneic HLA genotypically identical BMT, GvHD occurs in 15-35% of all cases, depending on the age of the recipient and the amount of T-cell depletion of the graft. The development of GvHD in recipients of HLA identical BM grafts reflects the role of mHag in BMT. Disparities for mHag between BM donor and recipient can evoke strong Major Histocompatibility Complex (MHC) restricted cytotoxic and proliferative T-cells responses, which can be measured in vitro (Goulmy 1997). Human mHags showed variable phenotype frequencies in the population and they are inherited in a Mendelian fashion independent of HLA (reviewed in Goulmy 1997). Recently, the chemical nature of the first series of murine and human mHags were identified (Goulmy 1996). It became clear that mHags are naturally processed fragments of intracellular proteins that associate with MHC molecules. With regard to the human mHags two H-Y mHags were identified as peptides from the SMCY protein (Wang 1995, Meadows 1997), the non-Y linked HA-2 antigen consists of nine residues and is most probably encoded by an as yet unidentified member of the non-filament forming class I myosin family (den Haan 1995). The human genes encoding mHags- with exception of the gene encoding the male specific mHags SMCY-have not been identified, yet. With the identification of HA-1, the first human autosomal mH gene was recognized (den Haan 1998). The mHag HA-1 was found to be a nonapeptide with the amino acid residues VLHDDLLEA, derived from an allele of the partially sequenced KIAA0223 cDNA (GENBANK Acc. No. D86976). The HA-1 allelic counterpart found in the data base, VLRDDLLEA, differs in one amino acid, i.e. R, from HA-1 antigen (den Haan 1998). Based on the KIAA0223 cDNA information, we subsequently designed HA-1 allele specific primers for RT-PCR. The amplification products correlated exactly with the HA-1 phenotype determined by HA-1 specific cytotoxic T-cells. Exploring the allele specific primers on genomic DNA however failed. Isolation and sequencing of cosmid DNA encoding the HA-1 sequence identified an intron present in the middle of the HA-1 coding sequence.

DETAILED DESCRIPTION OF THE INVENTION

It is an aim of the present invention to provide a method for genomic typing of the Minor Histocompatibility Antigen HA-1. Said method may be based on sequence-specific amplification, or on sequence-specific hybridization. It is therefore an aim of the present invention to elucidate the genomic structure of the HA-1 locus. It is an aim of the invention to provide primers and probes enabling said method for genomic typing. It is also an aim to provide kits enabling said method for genomic typing.

According to one embodiment, the present invention relates to a method for genomic typing of alleles of the Minor Histocompatibility Antigen HA-1 in a sample, with said method comprising:

a) contacting the genomic polynucleic acids in the sample with at least one pair of primers, whereby the 5'- and/or the 3'-primer of said at least one pair of primers specifically hybridize to target regions comprising polymorphic nucleotides in said alleles, and performing an amplification reaction;
b) for each of said at least one pair of primers detecting whether or not in step a) an amplification product is formed;
c) inferring from the result of step b) which HA-1 allele is present in said sample.

According to a preferred embodiment, the present invention relates to a method as described above, further characterized in that said alleles of the Minor Histocompatibility Antigen HA-1 are the H allele and the R allele (den Haan et al., 1998).

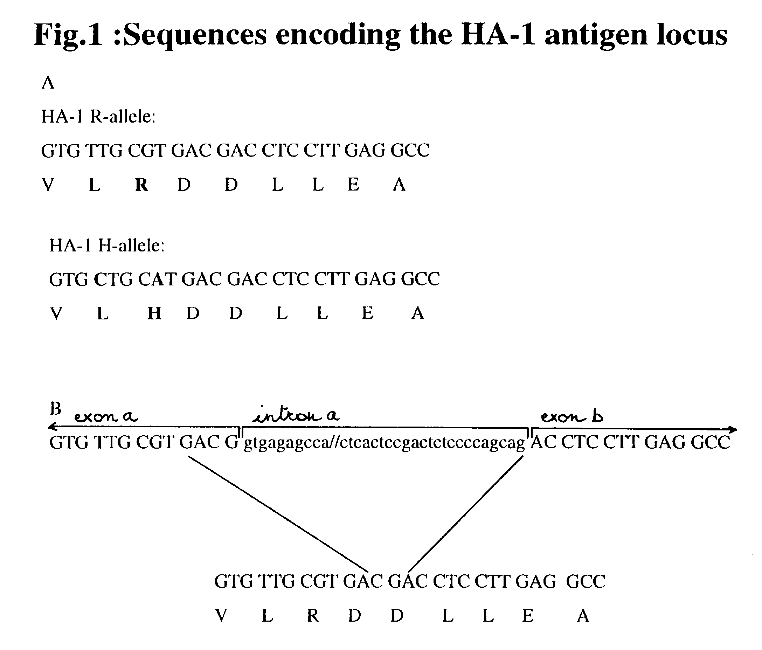

A method for typing of the HA-1 antigen by RT-PCR has been disclosed (den Haan et al., 1998). However, the present invention teaches that, unexpectedly, the primers used in the RT-PCR method, do not lead to amplification of a specific polynucleic acid fragment when genomic DNA is used as a template. To solve this problem, the present invention discloses the genomic structure of the HA-1 locus. As explained in Example 2, analysis of the genomic structure shows that the HA-1 peptide is encoded by two exons (figure 1). A splice donor site is located four nucleotides after the polymorphic codon in the HA-1 coding sequence. Therefore, setting up a method for genomic typing of the HA-1 antigen requires sequence information of the intron interrupting the HA-1 coding sequence. This sequence information is provided by the present invention and is shown below as SEQ ID NO 1.

gtg aga gcc acg ggg aca ccg agg cct ggg tgg aag aca gag cca gac cca agg gag gat gga ggg agg

gac ttg ggg agg ctc aga agg gag gga ggc tca gat ggc agg gag ggc tgt gtg gaa gag gcc atg aca gct

aag gct ctg agg gat gtg tag gag ttt ggt ggg gga gtc cct gag cgt aca ctg gct caa gag ggt gcc cac ttt

att ttt ttt aaa gga tct gat ggc aat tag gag gga aag gca gag gaa atg tcc cat gca cag gct cag aaa cac

gga aac aga gaa tgc att tgg ggg cca agg tgt ggg gtg ccg ctg gtg tag gat gaa ggc atg aca acg cca

ggc aga agg gca at     SEQ ID NO 1

This sequence represents part of the interrupting intron (indicated as intron a in figure 1), the first nucleotide of this sequence being the first nucleotide of intron a. The present invention thus also relates to an isolated polynucleic acid identified by SEQ ID NO 1, or an isolated polynucleic acid displaying at least 80%, or at least 90%, or at least 95%, or at least 99% sequence homology to SEQ ID NO 1, or any fragment of said polynucleic acids that can be used as a primer or as a probe.

Sequence information corresponding to another part of intron a, more particularly the part which is situated in front of exon b (figure 1) has been disclosed in the EMBL database under accession number AC004151. However, this sequence is not suitable for the design of primers for the above-mentioned method, since the length of the amplified fragment would lower the efficacy of the amplification reaction.

For detection of the amplification product mentioned in step b above, different methods known in the art, may be used. One method consists of subjecting the mixture obtained after the amplification reaction to gel electrophoresis and visually detecting the amplification product after nucleic acid staining. Alternatively, the amplification produet may be labeled, for instance by using labeled primers, and may be captured on a solid support, for instance by hybridization, and maybe detected on the solid support. It is clear, however, that other detection methods are also within the scope of the present invention.

According to a more preferred embodiment, the present invention relates to a method as indicated above, further characterized in that:

said at least one pair of primers comprises a 5'-primer that specifically hybridizes to a target region comprising the nucleotides at position 4 or at positions 4 and 8 in the HA-1 allele, or
said at least one pair of primers comprises a 3'-primer that specifically hybridizes to a target region comprising the nucleotides at position 8 or at positions 4 and 8 in the HA-1 allele, with said positions being indicated in figure 1.

According to an even more preferred embodiment, the present invention relates to a method as indicated above, further characterized in that:

said 5'-primer is combined with a 3'-primer specifically hybridizing to a target region in intron a, and/or
said 3'-primer is combined with a 5'-primer specifically hybridizing to a target region in exon a,
with intron a and exon a being indicated in figure 1.

According to this embodiment, said target region in intron a is ideally located in the sequence identified by SEQ ID NO 1, as explained above. Also the target region of said 3'-primer that is combined with a 5'-primer specifically hybridizing to a target region in exon a, will necessarily overlap with the sequence identified by SEQ ID NO 1.

According to an even more preferred embodiment, the present invention relates to a method as indicated above, further characterized in that the primers are chosen from Table 1:

| Name | Sequence (5' to 3') | SEQ ID NO |
|---|---|---|
| Set 1 | | |
| C-forward | GTGCTGCCTCCTGGACACTG | 2 |
| H-reverse | TGGCTCTCACCGTCATGCAG | 3 |
| R-reverse | TGGCTCTCACCGTCACGCAA | 4 |
| Set 2 | | |
| C-reverse | GCATTCTCTGTTTCCGTGTT | 5 |
| H-forward | CTTAAGGAGTGTGTGCTGCA | 6 |
| R-forward | CTTAAGGAGTGTGTGTTGCG | 7 |

*Table 1* Sequence of the primers used for genomic typing of HA-1 alleles by sequence-specific amplification.

Set 1 consists of a common 5'-primer (forward) and two different 3'-primers (reverse), one for the H allele and one for the R allele. The target region of the common 5'-primer is located in exon

a. The target region of the 3'-primers comprises the polymorphic nucleotides at positions 4 and 8 in the HA-1 coding sequence (figure 1) and partly overlaps with the sequence identified by SEQ ID NO 1. Set 2 consists of a common 3'-primer and two different 5'-primers. The target region of the common primer is located in the sequence identified by SEQ ID NO 1, whereas the target regions of the 5'-primers are located in exon a and comprise the polymorphic nucleotides at positions 4 and 8. Example 3 shows a genomic typing experiment making use of these primer sets.

According to another preferred embodiment, the present invention relates to a diagnostic kit for genomic typing of alleles of the Minor Histocompatibility Antigen HA-1 according to any of the methods indicated above, with said kit comprising:

a) at least one primer according to any of the methods indicated above;
b) optionally, an enzyme and/or reagents enabling the amplification reaction;
c) optionally, means enabling detection of the amplified products.

According to another preferred embodiment, the present invention relates to a method for genomic typing of alleles of the Minor Histocompatibility Antigen HA-1 in a sample, with said method comprising:

a) amplifying a fragment of said alleles, with said fragment comprising at least one polymorphic nucleotide, by use of at least one pair of primers specifically hybridizing to conserved target regions in said alleles;
b) hybridizing the amplified product of step a) to at least one probe specifically hybridizing to a target region comprising one or more polymorphic nucleotides in said allele;
c) inferring from the result of step b) which HA-1 allele is present in said sample.

According to a more peferred embodiment, the present invention relates to a method as indicated above, further characterized in that said alleles of the Minor Histocompatibility Antigen HA-1 are the H allele and the R allele.

According to an even more preferred embodiment, the present invention relates to a method as indicated above, further characterized in that said at least one pair of primers comprises a 5'-primer specifically hybridizing to a conserved target region in exon a and/or a 3'-primer specifically hybridizing to a conserved target region in intron a, with exon a and intron a being indicated in figure 1.

Ideally, the target region of said 3'-primer is located in the sequence identified as SEQ ID NO 1.

Obviously, the target region of said 3'-primer may also be located downstream of this sequence, i.e. in intron a, intron b, exon b, or even downstream of exon b, but the efficacy of the amplification reaction is likely to be lower as the amplified fragment becomes longer.

According to an even more preferred embodiment, the present invention relates to a method as indicated above, further characterized in that said at least one probe specifically hybridizes to a target region comprising the nucleotides at position 4 and/or 8 in the HA-1 allele, with said positions being indicated in figure 1.

According to an even more preferred embodiment, the present invention relates to a method as indicated above, further characterized in that said primers and/or said probes are chosen from Table 2.

| Name | Sequence (5' to 3') | SEQ ID NO |
|---|---|---|
| **Primers** | | |
| 5P1 (= C-forward) | GTGCTGCCTCCTGGACACTG | 2 |
| 3P1 | GCTGTCATGGCCTCTTCCAC | 8 |
| 3P2 | GCATTCTCTGTTTCCGTGTT | 9 |
| 3P3 | GGCAGAGAGCCCTCGCAGCC | 10 |
| **Probes** | | |
| HA1-R1(1) | TGTGTGTTGCGTGACGGTG | 11 |
| HA1-R1(2) | TGTGTGTTGCGTGACGGT | 12 |
| HA1-R1(3) | GTGTGTTGCGTGACGGTG | 13 |
| HA1-H1(1) | TGTGTGCTGCATGACGGTG | 14 |
| HA1-H1(2) | TGTGTGCTGCATGACGGT | 15 |
| HA1-H1(3) | GTGTGCTGCATGACGGTG | 16 |

*Table 2 Sequence of the primers and probes used for genomic typing of HA-1 alleles by amplification and sequence-specific hybridization*

Primers 3P1 and 3P2 specifically hybridize to target regions in SEQ ID NO 1. The target region of primer 3P3 is located downstream of exon b. The probes of Table 2 all specifically hybridize to target regions overlapping with the exon a-intron a boundary. The probes with SEQ ID NO 11 to 16 have been optimized to function in combination at the same conditions in a LiPA assay (see below). The skilled man will recognize that the probes and primers with SEQ ID NO 2 to 16 may be adapted by addition or deletion of one or more nucleotides at their extremities. Such adaptations may be required if the conditions of amplification or hybridization are changed, or if the amplified material is RNA instead of DNA, as is the case in the NASBA system. Different techniques can be applied to perform the sequence-specific hybridization methods of the present invention. These techniques may comprise immobilizing the amplified HA-1 polynucleic acids on a solid support and performing hybridization with labelled oligonucleotide probes. Genomic polynucleic acids may also be immobilized on a solid support without prior amplification and subjected to hybridization. Alternatively, the probes may be immobilized on a solid support and hybridization may be performed with labelled HA-1 polynucleic acids, preferably after amplification. This technique is called reverse hybridization. A convenient reverse hybridization technique is the line probe assay (LiPA). This assay uses oligonucleotide probes immobilized as parallel lines on a solid support strip (Stuyver et al., 1993). It is to be understood that any other technique for genomic typing of HA-1 alleles is also covered by the present invention.

It is clear that the present invention also relates to any of the primers with SEQ ID NO 2 to 10 and to any of the probes with SEQ ID NO 11 to 16, with said primers and said probes being for use in a method for genomic typing of alleles of the Minor Histocompatibility Antigen HA-1.

According to another preferred embodiment, the present invention relates to a diagnostic kit for genomic typing of alleles of the Minor Histocompatibility Antigen HA-1 according to any of the sequence-specific hybridization methods indicated above, with said kit comprising:

  a) at least one primer according to any of the methods indicated above;
  b) at least one probe according to any of the methods indicated above;
  c) optionally, an enzyme and/or reagents enabling the amplification reaction, and/or reagents enabling the hybridization reaction.

DEFINITIONS

The following definitions and explanations will permit a better understanding of the present invention.

The target material in the samples to be analysed will be genomic DNA or amplified versions thereof. These molecules are in this application also termed "polynucleic acids".

Well-known extraction and purification procedures are available for the isolation of RNA or DNA from a sample (e.g. in Sambrook et al.,1989).

A "polymorphic nucleotide" refers to a nucleotide of the sequence of a given HA-1 allele that differs from at least one of the nucleotides that are found at the corresponding position in other HA-1 alleles.

The term "typing" of an HA-1 allele refers to identification of the allele, i.e. detection of the allele and discrimination of the allele from other HA-1 alleles.

The term "probe" according to the present invention refers to a single-stranded oligonucleotide which is designed to specifically hybridize to HA-1 polynucleic acids. Preferably, the probes of the invention are about 5 to 50 nucleotides long, more preferably from about 10 to 30 nucleotides. Particularly preferred lengths of probes include 10, 11, 12, 13, 14, 15, 16, 17, 18, 19, 20, 21, 22, 23, 24, 25, 26, 27, 28, 29 or 30 nucleotides. The nucleotides as used in the present invention may be ribonucleotides, deoxyribonucleotides and modified nucleotides such as inosine or nucleotides containing modified groups which do not essentially alter their hybridization characteristics.

The term "primer" refers to a single stranded oligonucleotide sequence capable of acting as a point of initiation for synthesis of a primer extension product which is complementary to the nucleic acid strand to be copied. The length and the sequence of the primer must be such that they allow to prime the synthesis of the extension products. Preferably the primer is about 5-50 nucleotides long. Specific length and sequence will depend on the complexity of the required DNA or RNA targets, as well as on the conditions at which the primer is used, such as temperature and ionic strength. It is to be understood that the primers of the present invention may be used as probes and *vice versa*, provided that the experimental conditions are adapted.

The expression "suitable primer pair" in this invention refers to a pair of primers allowing specific amplification of a HA-1 polynucleic acid fragment.

The term "target region" of a probe or a primer according to the present invention is a sequence within the HA-1 polynucleic acids to which the probe or the primer is completely complementary or partially complementary (i.e. with some degree of mismatch). It is to be understood that the complement of said target sequence is also a suitable target sequence in some cases.

"Specific hybridization" of a probe to a target region of the HA-1 polynucleic acids means that said probe forms a duplex with part of this region or with the entire region under the experimental conditions used, and that under those conditions said probe does not form a duplex with other regions of the polynucleic acids present in the sample to be analysed.

"Specific hybridization" of a primer to a target region of the HA-1 polynucleic acids means that, during the amplification step, said primer forms a duplex with part of this region or with the entire region under the experimental conditions used, and that under those conditions said primer does not form a duplex with other regions of the polynucleic acids present in the sample to be analysed. It is to be understood that "duplex" as used hereby, means a duplex that will lead to specific amplification.

"Specific amplification" of a fragment of the HA-1 polynucleic acids means amplification of the fragment for which the primers were designed, and not of any other fragment of the polynucleic acids present in a sample.

The fact that amplification primers do not have to match exactly with the corresponding target sequence in the template to warrant proper amplification is amply documented in the literature (Kwok et al., 1990). However, when the primers are not completely complementary to their target sequence, it should be taken into account that the amplified fragments will have the sequence of the primers and not of the target sequence. Primers may be labelled with a label of choice (e.g. biotine). The amplification method used can be either polymerase chain reaction (PCR; Saiki et al., 1988), ligase chain reaction (LCR; Landgren et al, 1988; Wu & Wallace, 1989; Barany, 1991), nucleic acid sequence-based amplification (NASBA; Guatelli et al, 1990; Compton, 1991), transcription-based amplification system (TAS; Kwoh et al, 1989), strand displacement amplification (SDA; Duck, 1990) or amplification by means of Qβ replicase (Lomeli et al., 1989) or any other suitable method to amplify nucleic acid molecules known in the art. Probe and primer sequences are represented throughout the specification as single stranded DNA oligonucleotides from the 5' to the 3' end. It is obvious to the man skilled in the art that any of the below-specified probes can be used as such, or in their complementary form, or in their RNA form (wherein T is replaced by U).

The probes according to the invention can be prepared by cloning of recombinant plasmids containing inserts including the corresponding nucleotide sequences, if need be by excision of the latter from the cloned plasmids by use of the adequate nucleases and recovering them, e.g. by fractionation according to molecular weight. The probes according to the present invention can also be synthesized chemically, for instance by the conventional phospho-triester method.

The oligonucleotides used as primers or probes may also comprise nucleotide analogues such as phosphorothiates (Matsukura et al, 1987), alkylphosphorothiates (Miller et al., 1979) or peptide nucleic acids (Nielsen et al., 1991; Nielsen et al., 1993) or may contain intercalating agents (Asseline et al., 1984). As most other variations or modifications introduced into the original DNA sequences of the invention these variations will necessitate adaptions with respect to the conditions under which the oligonucleotide should be used to obtain the required specificity and sensitivity. However the eventual results of hybridization will be essentially the same as those obtained with the unmodified oligonucleotides. The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, $NH_2$ groups, SH groups, carboxylic groups, or coupling with biotin, haptens or proteins.

The term "labelled" refers to the use of labelled nucleic acids. Labelling may be carried out by the use of labelled nucleotides incorporated during the polymerase step of the amplification such as illustrated by Saiki et al. (1988) or Bej et al. (1990) or labelled primers, or by any other method known to the person skilled in the art. The nature of the label may be isotopic ($^{32}P$, $^{35}S$, etc.) or non-isotopic (biotin, digoxigenin, etc.).

The "biological sample" may be for instance blood, mouth swab or any other sample comprising genomic DNA.

For designing probes with desired characteristics, the following useful guidelines known to the person skilled in the art can be applied.

Because the extent and specificity of hybridization reactions such as those described herein are affected by a number of factors, manipulation of one or more of those factors will determine the exact sensitivity and specificity of a particular probe, whether perfectly complementary to its target or not. The importance and effect of various assay conditions are explained further herein.

**The stability of the [probe : target] nucleic acid hybrid should be chosen to be compatible with the assay conditions. This may be accomplished by avoiding long AT-rich sequences, by terminating the hybrids with G:C base pairs, and by designing the probe with an appropriate Tm. The beginning and end points of the probe should be chosen so that the length and %GC result in a Tm about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be more stable at higher temperatures.

**Conditions such as ionic strength and incubation temperature under which a probe will be used should also be taken into account when designing a probe. It is known that the degree of hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tm. In general, optimal hybridization for synthetic oligonucleotide probes of about 10-50 bases in length occurs approximately 5°C below the melting temperature for a given duplex. Incubation at temperatures below the optimum may allow mismatched base sequences to hybridize and can therefore result in reduced specificity.

**It is desirable to have probes which hybridize only under conditions of high stringency. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. The degree of stringency is chosen such as to maximize the difference in stability between the hybrid formed with the target and the non-target nucleic acid.

**Regions in the target DNA or RNA which are known to form strong internal structures inhibitory to hybridization are less preferred. Likewise, probes with extensive self-complementarity should be avoided. As explained above, hybridization is the association of two single strands of complementary nucleic acids to form a hydrogen bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

**Standard hybridization and wash conditions are disclosed in the Materials & Methods section of the Examples. Other conditions are for instance 3X SSC (Sodium Saline Citrate), 20% deionized FA (Formamide) at 50°C. Other solutions (SSPE (Sodium saline phosphate EDTA), TMAC (Tetramethyl ammonium Chloride), etc.) and temperatures can also be used provided that the specificity and sensitivity of the probes is maintained. When needed, slight modifications of the probes in length or in sequence have to be carried out to maintain the specificity and sensitivity required under the given circumstances.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

FIGURE AND TABLE LEGENDS

Table 1

Sequence of the primers used for genomic typing of HA-1 alleles by sequence-specific amplification.

Table 2

Sequence of the primers and probes used for genomic typing of HA-1 alleles by amplification and sequence-specific hybridization.

Table 3

Cellular and genomic typing for HA-1 in three HLA-A*0201 positive families.

Figure 1

Sequences and genomic structure of the HA-1 locus. Figure 1a, coding sequences of the H and R alleles of HA-1. Bold characters indicate the polymorphic nucleotides. Figure 1b, exon-intron boundaries of the HA-1 locus. Exon sequences are shown in uppercase, intron sequences in lowercase.

Figure 2

Genomic typing of HA-1 alleles in clinical samples. Genomic typing was performed by sequence-specific amplification, by use of the two primer sets of Table 1. The two upper fragments in the gel originate from the H-allele, the two lower fragments from the R-allele.

EXAMPLES

Example 1. Materials and Methods

*Cell culture and isolation of genomic DNA:*

Genomic DNA was isolated from frozen peripheral blood lymphocytes (PBL) with the High Pure Template Purification Kit from Boebringer Mannheim according to the manufacturers description. EBV-transformed LCLs cells were cultured in RPMI-1640 containing 3mM L-glutamine and 10% FCS. For DNA isolation the cells were harvested, washed twice with phosphate buffered saline (PBS), resuspended in 200 $\mu$l and kept at -20C until use. For each DNA isolation $2x10^6$ cells were used.

*Genomic PCR:*

For each PCR reaction 100-200 ng of genomic DNA were used. Amplifications were performed with 20 pmol of each primer in 100 μl of 10mM Tris/HCl (pH8.4) buffer, containing 50mM KCl, 4mM MgCl2, 0.06mg/ml BSA, 0.5 mM dNTP's and 2.5 units Taq polymerase (Roche Molecular Systems, Brancheburg, New Jersey). All reactions started with a denaturation step of 5 min. at 95°C. The cycling conditions for all primer combinations were 95°C for 1 min. and 65°C for 1 min. for ten cycles. Followed by 20 cycles at 95°C for 1 min., 62°C for 1 min. , 72°C for 1 min., and an extension of the last step for 5 min. at 72°C.

*Isolation of cosmid DNA:*

For the large scale isolation of cosmid DNA, 1l of LB-medium (20μg/ml Ampicillin) was inocculated and grown overnight at 37°C with vigorous shaking (300rpm). The cosmid DNA was isolated by using the low-copy number plasmid isolation protocol of the Quiagen Plasmid Purification Kit according to the manufacturers description. This isolation method yielded on average in 500 μg of purified cosmid DNA.

*Sequencing of cosmid DNA:*

For each sequencing reaction, 10μg of cosmid DNA. The sequencing reactions were performed with the T7 Sequencing kit (Pharmacia Biotech).

*mHag HA-1 specific CTL:*

EBV-transformed-LCLs were tested with HA-1 specific CTLs. Reactivity was determine by a chromium release assay. $^{51}$Cr labelled target cells ($3x10^3$) were co-incubated with serial dilutions of effector T-cells in 96 well round bottom microtiter plates (Costar 3799). After 4 hours at 37°C cell free supernatants were harvested for gamma counting. The percent specific lysis was calculated as follows : 5 specific lysis = (experimental release-spontanous release)/ (maximal release-spontanous release)x100% . Spontanous release and maximum release are the chromium release of target cells in culture medium alone and in culture medium containing 1% Triton-X 100, respectively.

Example 2 The HA-1 peptide is encoded by two exons

For the determination of the genomic structure surrounding the HA-1 peptide a cosmid library derived from human male PBLs was screened. The 312bp cDNA fragment encoding the HA-1 peptide was used as probe for screening (den Haan et al., 1998). Three overlapping cosmids were isolated. The cosmid pTCF-HA-1 containing the R-allele was partially sequenced. The sequence reaction revealed a splice donor site four nucleotides after the HA-1 polymorphic codon. A splice acceptor site could be identified in front of the second exon coding for the HA-1 peptide (Fig. 1). Thus, the HA-1 peptide sequence is encoded by two exons.

Example 3 Allele-specific PCR on genomic DNA

For the genomic allele-specific typing two different primer sets were designed. Both sets do contain a common primer and one specific for either the HA-1 H-allele or R-allele (table 1). The common primer of set 1 is derived from the exon encoding the first four amino acids of the HA-1 peptide. The H/R primers contain intronic sequences , the splice donor site and the allele specific part of the exon sequence. Set 2 consists of a common primer derived from the intron identified in pTCF-HA-1 and exon derived primers covering the H- and R-allele. Amplification with primer set 1 resulted in a 190bp fragment, primer set 2 gave a 331bp fragment. Both primer sets showed the expected length of fragments and are suitable for genomic typing. Because the primers were chosen in such a way, that they should amplify the DNA under identical PCR conditions, a combination of both primer sets can be used in the same PCR reaction. In this case, a third fragment of 535bp was observed due the amplification of the DNA between the two different common primers (data not shown).

Example 4 Family studies

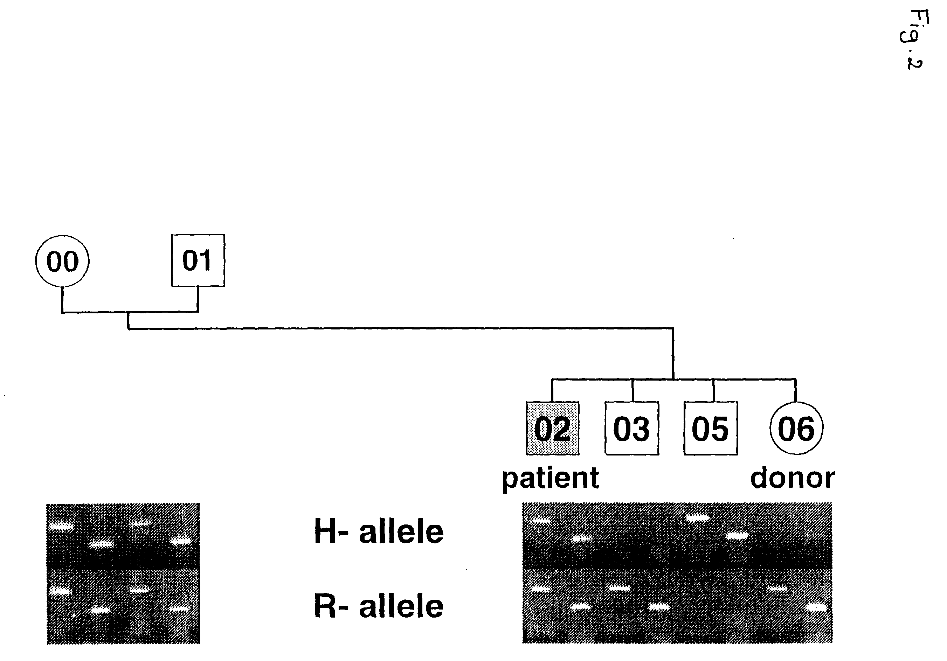

The feasibility of genomic typing was carried out on 24 members belonging to three HLA-A*0201 positive families. The results of the DNA typing was compared with the mHag HA-1 CTL typing (table 3) and showed an exact correlation. Figure 2 shows the genomic DNA analysis of the HA-1 locus in a representative family. The bone marrow donor (06) and recipient (02) were HLA-identical. The donor was homozygous for the R-allele. The recipient was heterozygous (H/R) and therefore presenting the HA-1 antigen at the cell surface. This mismatch resulted in GvHD, thus the T-cells of the donor reacted against the mHag of the recipient. In this familiy the donor and recipient were HLA-identical, but they had a mismatch in the HA-1 sequence. The same disparities for HA-1 could be observed in family 2. Again the donor (07) was homozygous for the R-allel and the patient (02) was heterozygous (H/R), resulting in GvHD. Family 3 represents the segregation of the HA-1 H-allele in three generations in an healthy family. The H-allele is derived from the grandfather (01) and is inherited by two generations. The grandmother (00) although HLA-A*0201 positive is homozygous for the R-allele. Their children (03, 04, and 05) are all heterozygous for the HA-1 locus. The child 04 married individual 34 who is HLA-A*0201 positive, but homozygous for the R-allele. From their offspring, only 84 inherited the HA-1 H-allele from the grandfather. The other grandchildren (82, 83 and 85) are HA-1 R homozygous.

Table 3

| Cellular and genomic typing for HA-1 in three HLA-A*0201 positive families | | |
|---|---|---|
| Family1 | CML analysis | PCR analysis |
| 00 | + | H/R |
| 01 | + | H/R |
| 02* | +. | H/R |
| 03 | - | R/R |
| 05 | + | H/H |
| 06* | - | R/R |
| Family2 | | |
| 00 | - | R/R |
| 01 | + | H/R |
| 02* | + | H/R |
| 04 | + | H/R |
| 06 | + | H/R |
| 07* | - | R/R |
| 09 | - | R/R |
| 10 | + | H/R |
| Family3 | | |
| 00 | - | R/R |
| 01 | + | H/R |
| 03 | + | H/R |
| 04 | + | H/R |
| 05 | + | H/R |
| 34 | - | R/R |
| 82 | - | R/R |
| 83 | - | R/R |
| 84 | + | H/R |
| 85 | - | R/R |

Example 5 Typing of HA-1 alleles by the LiPA method

The following method for typing of the HA-1 alleles H and R in a sample, is based on the LiPA technology (Stuyver et al. 1993). For each PCR reaction 100-200 ng of genomic DNA is used. Amplifications are performed with 20 pmol of each primer in 100 μl of 10mM Tris/HCl (pH8.4) buffer, containing 50mM KCl, 4mM MgCl2, 0.06mg/ml BSA, 0.5 mM dNTP's and 2.5 units Taq polymerase (Roche Molecular Systems, Brancheburg, New Jersey). All reactions start with a denaturation step of 5 min. at 95°C. The cycling conditions for all primer combinations are 95°C for 1 min. and 65°C for 1 min. for ten cycles. Followed by 20 cycles at 95°C for 1 min., 62°C for 1 min. , 72°C for 1 min., and an extension of the last step for 5 min. at 72°C. The HA-1 alleles are subsequently typed by a reverse hybridization step to oligonucleotide probes that are immobilized on a nitro-cellulose strip. Probes specifically hybridizing to the R-allele are for instance HA1-R1(1) (SEQ ID NO 11), HA1-R1(2) (SEQ ID NO 12) and HA1-R1(3) (SEQ ID NO 13). Probes specifically hybridizing to the H-allele are for instance HA1-H1(1) (SEQ ID NO 14), HA1-H1(2) (SEQ ID NO 15) and HA1-H1(3) (SEQ ID NO 16). The hybridization is performed in 5x SSPE, 0.5% SDS at 56°C for 30 min. A stringent washing step is carried out in 2x SSPE, 0.1% SDS at 56°C for 10 min.

REFERENCES

Asseline U, Delarue M, Lancelot G, Toulme F, Thuong N (1984) Nucleic acid-binding molecules with high affinity and base sequence specificity : intercalating agents covalently linked to oligodeoxynucleotides. Proc. Natl. Acad. Sci. USA 81(11):3297-301.

Bej A, Mahbubani M, Miller R, Di Cesare J, Haff L, Atlas R. (1990) Multiplex PCR amplification and immobilized capture probes for detection of bacterial pathogens and indicators in water. Mol Cell Probes 4:353-365.

Compton J. (1991) Nucleic acid sequence-based amplification. Nature 350: 91-92.

den Haan J, Meadows L, Wang W, Pool J, Blokland E, Bishop T, Reinhardus C, Shabanowitz J, Offringa R, Hunt D, Engelhard V and Goulmy E (1998) The minor histocompatibility antigen HA-1: a diallelic gene with a single amino acid polymorphism. Science 279, 1054-1057.

Duck P. (1990) Probe amplifier system based on chimeric cycling oligonucleotides. Biotechniques 9: 142-147.

Goulmy E. (1996) Human minor histocompatibility antigens. Curr Opin Immunol. 8: 75-81.

Goulmy E. (1997) Human minor histocompatibility antigens: new concepts for marrow transplantation and adoptive immunotherapy. Immunol Rev. 157: 125-140.

Guatelli J, Whitfield K, Kwoh D, Barringer K, Richman D, Gengeras T. (1990) Isothermal, in vitro amplification of nucleic acids by a multienzyme reaction modeled after retroviral replication. Proc Natl Acad Sci USA 87: 1874-1878.

Kwoh D, Davis G, Whitfield K, Chappelle H, Dimichele L, Gingeras T. (1989)Transcription-based amplification system and detection of amplified human immunodeficiency virus type 1 with a bead-based sandwich hybridization format. Proc Natl Acad Sci USA 86: 1173-1177.

Kwok S, Kellogg D, McKinney N, Spasic D, Goda L, Levenson C, Sinisky J. (1990) Effects of primer-template mismatches on the polymerase chain reaction: Human immunodeficiency virus type 1 model studies. Nucl. Acids Res. 18: 999.

Landgren U, Kaiser R, Sanders J, Hood L. (1988) A ligase-mediated gene detection technique. Science 241:1077-1080.

Lomeli H, Tyagi S, Pritchard C, Lisardi P, Kramer F. (1989) Quantitative assays based on the use of replicatable hybridization probes. Clin Chem 35: 1826-1831.

Matsukura M, Shinozuka K, Zon G, Mitsuya H, Reitz M, Cohen J, Broder S (1987) Phosphorothioate analogs of oligodeoxynucleotides : inhibitors of replication and cytopathic effects of human immunodeficiency virus. Proc. Natl. Acad. Sci. USA 84(21): 7706-10.

Miller P, Yano J, Yano E, Carroll C, Jayaram K, Ts'o P (1979) Nonionic nucleic acid analogues. Synthesis and characterization of dideoxyribonucleoside methylphosphonates. Biochemistry 18(23):5134-43.

Mullis K, Faloona F., Scharf S. *et al.* Specific enzymatic amplification of DNA in vitro: the polymerase chain reaction (1986). *Gold Spring Harb. Symp. Quant. Biol.* **1**: 263-273.

Mullis K.B. and Faloona F.A. Specific synthesis of DNA in vitro via a polymerase-catalyzed chain reaction (1987). *Methods Enzymol.* **155**: 335-350.

Nielsen P, Egholm M, Berg R, Buchardt O (1991) Sequence-selective recognition of DNA by strand displacement with a thymine-substituted polyamide. Science 254(5037):1497-500.

Nielsen P, Egholm M., Berg R, Buchardt O (1993) Sequence specific inhibition of DNA restriction enzyme cleavage by PNA. Nucleic-Acids-Res. 21(2): 197-200.

Santamaria P., Boyce J.M., Lindstrom A.L. *et al.* HLA class II "typing": direct sequencing of DRB, DQB and DQA genes (1992). *Hum. Immunol.* **33**: 69-81.

Saiki R.K., Bugawan T.L., Horn G.T. *et al.* Analysis of enzymatically amplified beta-globin and HLA-DQ alpha DNA with allele-specific oligonucleotide probes (1986). *Nature* **324**: 163-166.

Saiki R.K., Walsh P.S., Levenson, C.H. and Erlich H.A. Genetic analysis of amplified DNA with immobilized sequence-specific oligonucleotide probes (1989). *Proc. Natl. Acad Sci. USA* **86:** 6230-6234.

Sambrook J, Fritsch E, Maniatis T (1989) Molecular cloning: a laboratory manual. Cold Spring Harbor Laboratory Press, Cold Spring Harbor, NY.

Spencer Wells R, Parham P; HLA class I genes: structure and diversity. Chapter 4. HLA and MHC: genes, molecules and function. 1996 BIOS Scientific Publishers Ltd, Oxford.

Stuyver L, Rossau R, Wyseur A et al (1993) Typing of hepatitis C virus isolates and characterization of new subtypes using a line probe assay. J. Gen. Virol. 74: 1093-1102

Terasaki P.H. and McClelland, J.D. Microdoplet assay of human serum cytotoxins.(1964) *Nature* **204**: 998-1007.

Wu D, Wallace B. (1989) The ligation amplification reaction (LAR) - amplification of specific DNA sequences using sequential rounds of template-dependent ligation. Genomics 4:560-569.

## Claims

1. Method for genomic typing of alleles of the Minor Histocompatibility Antigen HA-1 in a sample, with said method comprising:

    a) contacting the genomic polynucleic acids in the sample with at least one pair of primers, whereby the 5'- and/or the 3'-primer of said at least one pair of primers specifically hybridize to target regions comprising polymorphic nucleotides in said alleles, and performing an amplification reaction;
    b) for each of said at least one pair of primers detecting whether or not in step a) an amplification product is formed;
    c) inferring from the result of step b) which HA-1 allele is present in said sample.

2. Method according to claim 1, further characterized in that said alleles of the Minor Histocompatibility Antigen HA-1 are the H allele and the R allele.

3. Method according to any of claims 1 and 2, further characterized in that:

> said at least one pair of primers comprises a 5'-primer that specifically hybridizes to a target region comprising the nucleotides at position 4 or at positions 4 and 8 in the HA-1 allele, or
> said at least one pair of primers comprises a 3'-primer that specifically hybridizes to a target region comprising the nucleotides at position 8 or at positions 4 and 8 in the HA-1 allele, with said positions being indicated in figure 1.

4. Method according to claim 3, further characterized in that:

> said 5'-primer is combined with a 3'-primer specifically hybridizing to a target region in intron a, and/or
> said 3'-primer is combined with a 5'-primer specifically hybridizing to a target region in exon a,
> with intron a and exon a being indicated in figure 1.

5. Method according to any of claims 1 to 4, further characterized in that the primers are chosen from the following list:

> SEQ ID NO 2, SEQ ID NO 3, SEQ ID NO 4, SEQ ID NO 5, SEQ ID NO 6, SEQ ID NO 7.

6. Method for genomic typing of alleles of the Minor Histocompatibility Antigen HA-1 in a sample, with said method comprising:

> a) amplifying a fragment of said alleles, with said fragment comprising at least one polymorphic nucleotide, by use of at least one pair of primers specifically hybridizing to conserved target regions in said alleles;
> b) hybridizing the amplified product of step a) to at least one probe specifically hybridizing to a target region comprising one or more polymorphic nucleotides in said allele;
> c) inferring from the result of step b) wich HA-1 allele is present in said sample.

7. Method according to claim 6, further characterized in that said alleles of the Minor Histocompatibility Antigen HA-1 are the H allele and the R allele.

8. Method according to any of claims 6 and 7, further characterized in that said at least one pair of primers comprises a 5'-primer specifically hybridizing to a conserved target region in exon a and/or a 3'-primer specifically hybridizing to a conserved target region in intron a, with exon a and intron a being indicated in figure 1.

9. Method according to any of claims 6 to 8, further characterized in that said at least one probe specifically hybridizes to a target region comprising the nucleotides at position 4 and/or 8 in the HA-1 allele, with said positions being indicated in figure 1.

10. Method according to any of claims 6 to 9, further characterized in that: said primers are chosen from the following list:

> SEQ ID NO 2, SEQ ID NO 8, SEQ ID NO 9, SEQ ID NO 10, and/or
> said probes are chosen from the following list:
> SEQ ID NO 11, SEQ ID NO 12, SEQ ID NO 13, SEQ ID NO 14, SEQ ID NO 15, SEQ ID NO 16

11. A primer for use in a method according to any of claims 1 to 10 for genomic typing of alleles of the Minor Histocompatibility Antigen HA-1.

12. A probe for use in a method according to any of claims 6 to 10 for genomic typing of alleles of the Minor Histocompatibility Antigen HA-1.

13. An isolated polynucleic acid identified by SEQ ID NO 1, or an isolated polynucleic acid displaying at least 80% sequence homology to SEQ ID NO 1, or any fragment of said polynucleic acids that can be used as a primer or as a probe.

14. A diagnostic kit for genomic typing of alleles of the Minor Histocompatibility Antigen HA-1 according to any of claims 1 to 5, with said kit comprising:

a) at least one primer according to any of claims 1 to 5;

b) optionally, an enzyme and/or reagents enabling the amplification reaction;

c) optionally, means enabling detection of the amplified products.

15. A diagnostic kit for genomic typing of alleles of the Minor Histocompatibility Antigen HA-1 according to any of claims 6 to 10, with said kit comprising:

a) at least one primer according to any of claims 6 to 10;

b) at least one probe according to any of claims 6 to 10;

c) optionally, an enzyme and/or reagents enabling the amplification reaction, and/or reagents enabling the hybridization reaction.

# Fig.1 :Sequences encoding the HA-1 antigen locus

A

HA-1 R-allele:

GTG TTG CGT GAC GAC CTC CTT GAG GCC

V    L    **R**    D    D    L    L    E    A

HA-1 H-allele:

GTG CTG CAT GAC GAC CTC CTT GAG GCC

V    L    **H**    D    D    L    L    E    A

B   *exon a*            *intron a*            *exon b*

GTG TTG CGT GAC G | gtgagagcca//ctcactccgactctccccagcag | AC CTC CTT GAG GCC

GTG TTG CGT GAC GAC CTC CTT GAG GCC

V    L    R    D    D    L    L    E    A

EP 0 965 648 A1

Fig. 2

EP 0 965 648 A1

**European Patent Office**

**EUROPEAN SEARCH REPORT**

Application Number

EP 98 87 0125

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | DEN HAAN J ET AL: "The minor histocompatibility antigen HA-1: A diallelic gene with a single amino acid polymorphism" SCIENCE, vol. 279, 13 February 1998, pages 1054-7, XP002083017 * the whole document * | 1-12,14, 15 | C12Q1/68 C12N15/12 |
| A | SIMPSON E ET AL: "Much ado about minor histocompatibility antigens" IMMUNOLOGY TODAY, vol. 19, no. 3, March 1998, pages 108-112, XP002083018 * page 111, paragraph 5 * | 1-12,14, 15 | |
| A | GOULMY E: "Minor histocompatibility antigens: From T cell recogition to peptide identification" HUMAN IMMUNOLOGY, vol. 54, no. 1, April 1997, pages 8-14, XP002083019 * page 11, paragraph 2 - paragraph 3 * | 1 | TECHNICAL FIELDS SEARCHED (Int.Cl.6)<br><br>C12Q |

The present search report has been drawn up for all claims

| Place of search | Date of completion of the search | Examiner |
|---|---|---|
| THE HAGUE | 5 November 1998 | Osborne, H |

EPO FORM 1503 03.82 (P04C01)

SEQUENCE LISTING

(1) GENERAL INFORMATION:

    (i) APPLICANT:
        (A) NAME: Innogenetics N.V.
        (B) STREET: Industriepark Zwijnaarde 7, box 4
        (C) CITY: Ghent
        (E) COUNTRY: Belgium
        (F) POSTAL CODE (ZIP): B-9052
        (G) TELEPHONE: 00 32 9 241 07 11
        (H) TELEFAX: 00 32 9 241 07 99

    (ii) TITLE OF INVENTION: Method for genomic typing of the Minor
        Histocompatibility Antigen HA-1.

    (iii) NUMBER OF SEQUENCES: 16

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

    (v) CURRENT APPLICATION DATA:
        APPLICATION NUMBER: EP 98870125.6

(2) INFORMATION FOR SEQ ID NO: 1:

    (i) SEQUENCE CHARACTERISTICS:
        (A) LENGTH: 377 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO


    (xi) SEQUENCE DESCRIPTION: SEQ ID NO: 1:

```
GTGAGAGCCA CGGGGACACC GAGGCCTGGG TGGAAGACAG AGCCAGACCC AAGGGAGGAT      60

GGAGGGAGGG ACTTGGGGAG GCTCAGAAGG GAGGGAGGCT CAGATGGCAG GGAGGGCTGT     120

GTGGAAGAGG CCATGACAGC TAAGGCTCTG AGGGATGTGT AGGAGTTTGG TGGGGGAGTC     180

CCTGAGCGTA CACTGGCTCA AGAGGGTGCC CACTTTATTT TTTTTAAAGG ATCTGATGGC     240

AATTAGGAGG GAAAGGCAGA GGAAATGTCC CATGCACAGG CTCAGAAACA CGGAAACAGA     300

GAATGCATTT GGGGGCCAAG GTGTGGGGTG CCGCTGGTGT AGGATGAAGG CATGACAACG     360

CCAGGCAGAA GGGCAAT                                                    377
```

(2) INFORMATION FOR SEQ ID NO: 2:

    (i) SEQUENCE CHARACTERISTICS:
        (A) LENGTH: 20 base pairs
        (B) TYPE: nucleic acid

```
        (C) STRANDEDNESS: single
        (D) TOPOLOGY: linear

    (ii) MOLECULE TYPE: DNA (genomic)

   (iii) HYPOTHETICAL: NO

    (iv) ANTI-SENSE: NO



    (xi) SEQUENCE DESCRIPTION: SEQ ID NO: 2:

GTGCTGCCTC CTGGACACTG                                     20

(2) INFORMATION FOR SEQ ID NO: 3:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

   (iii) HYPOTHETICAL: NO

    (iv) ANTI-SENSE: NO



    (xi) SEQUENCE DESCRIPTION: SEQ ID NO: 3:

TGGCTCTCAC CGTCATGCAG                                     20

(2) INFORMATION FOR SEQ ID NO: 4:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

   (iii) HYPOTHETICAL: NO

    (iv) ANTI-SENSE: NO



    (xi) SEQUENCE DESCRIPTION: SEQ ID NO: 4:

TGGCTCTCAC CGTCACGCAA                                     20

(2) INFORMATION FOR SEQ ID NO: 5:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear
```

(ii) MOLECULE TYPE: DNA (genomic)

(iii) HYPOTHETICAL: NO

(iv) ANTI-SENSE: NO


(xi) SEQUENCE DESCRIPTION: SEQ ID NO: 5:

GCATTCTCTG TTTCCGTGTT                                                    20

(2) INFORMATION FOR SEQ ID NO: 6:

        (i) SEQUENCE CHARACTERISTICS:
            (A) LENGTH: 20 base pairs
            (B) TYPE: nucleic acid
            (C) STRANDEDNESS: single
            (D) TOPOLOGY: linear

        (ii) MOLECULE TYPE: DNA (genomic)

        (iii) HYPOTHETICAL: NO

        (iv) ANTI-SENSE: NO


        (xi) SEQUENCE DESCRIPTION: SEQ ID NO: 6:

CTTAAGGAGT GTGTGCTGCA                                                    20

(2) INFORMATION FOR SEQ ID NO: 7:

        (i) SEQUENCE CHARACTERISTICS:
            (A) LENGTH: 20 base pairs
            (B) TYPE: nucleic acid
            (C) STRANDEDNESS: single
            (D) TOPOLOGY: linear

        (ii) MOLECULE TYPE: DNA (genomic)

        (iii) HYPOTHETICAL: NO

        (iv) ANTI-SENSE: NO


        (xi) SEQUENCE DESCRIPTION: SEQ ID NO: 7:

CTTAAGGAGT GTGTGTTGCG                                                    20

(2) INFORMATION FOR SEQ ID NO: 8:

        (i) SEQUENCE CHARACTERISTICS:
            (A) LENGTH: 20 base pairs
            (B) TYPE: nucleic acid
            (C) STRANDEDNESS: single
            (D) TOPOLOGY: linear

        (ii) MOLECULE TYPE: DNA (genomic)

        (iii) HYPOTHETICAL: NO

(iv) ANTI-SENSE: NO

(xi) SEQUENCE DESCRIPTION: SEQ ID NO: 8:

GCTGTCATGG CCTCTTCCAC                                         20

(2) INFORMATION FOR SEQ ID NO: 9:

    (i) SEQUENCE CHARACTERISTICS:
       (A) LENGTH: 20 base pairs
       (B) TYPE: nucleic acid
       (C) STRANDEDNESS: single
       (D) TOPOLOGY: linear

    (ii) MOLECULE TYPE: DNA (genomic)

    (iii) HYPOTHETICAL: NO

    (iv) ANTI-SENSE: NO

(xi) SEQUENCE DESCRIPTION: SEQ ID NO: 9:

GCATTCTCTG TTTCCGTGTT                                         20

(2) INFORMATION FOR SEQ ID NO: 10:

    (i) SEQUENCE CHARACTERISTICS:
       (A) LENGTH: 20 base pairs
       (B) TYPE: nucleic acid
       (C) STRANDEDNESS: single
       (D) TOPOLOGY: linear

    (ii) MOLECULE TYPE: DNA (genomic)

    (iii) HYPOTHETICAL: NO

    (iv) ANTI-SENSE: NO

(xi) SEQUENCE DESCRIPTION: SEQ ID NO: 10:

GGCAGAGAGC CCTCGCAGCC                                         20

(2) INFORMATION FOR SEQ ID NO: 11:

    (i) SEQUENCE CHARACTERISTICS:
       (A) LENGTH: 19 base pairs
       (B) TYPE: nucleic acid
       (C) STRANDEDNESS: single
       (D) TOPOLOGY: linear

    (ii) MOLECULE TYPE: DNA (genomic)

    (iii) HYPOTHETICAL: NO

    (iv) ANTI-SENSE: NO

(xi) SEQUENCE DESCRIPTION: SEQ ID NO: 11:

TGTGTGTTGC GTGACGGTG                                                      19

(2) INFORMATION FOR SEQ ID NO: 12:

    (i) SEQUENCE CHARACTERISTICS:
        (A) LENGTH: 18 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO: 12:

TGTGTGTTGC GTGACGGT                                                       18

(2) INFORMATION FOR SEQ ID NO: 13:

    (i) SEQUENCE CHARACTERISTICS:
        (A) LENGTH: 18 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO: 13:

GTGTGTTGCG TGACGGTG                                                       18

(2) INFORMATION FOR SEQ ID NO: 14:

    (i) SEQUENCE CHARACTERISTICS:
        (A) LENGTH: 19 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (xi) SEQUENCE DESCRIPTION: SEQ ID NO: 14:

```
TGTGTGCTGC ATGACGGTG                                                    19
```

(2) INFORMATION FOR SEQ ID NO: 15:

    (i) SEQUENCE CHARACTERISTICS:
       (A) LENGTH: 18 base pairs
       (B) TYPE: nucleic acid
       (C) STRANDEDNESS: single
       (D) TOPOLOGY: linear

   (ii) MOLECULE TYPE: DNA (genomic)

  (iii) HYPOTHETICAL: NO

   (iv) ANTI-SENSE: NO

   (xi) SEQUENCE DESCRIPTION: SEQ ID NO: 15:

```
TGTGTGCTGC ATGACGGT                                                     18
```

(2) INFORMATION FOR SEQ ID NO: 16:

    (i) SEQUENCE CHARACTERISTICS:
       (A) LENGTH: 18 base pairs
       (B) TYPE: nucleic acid
       (C) STRANDEDNESS: single
       (D) TOPOLOGY: linear

   (ii) MOLECULE TYPE: DNA (genomic)

  (iii) HYPOTHETICAL: NO

   (iv) ANTI-SENSE: NO

   (xi) SEQUENCE DESCRIPTION: SEQ ID NO: 16:

```
GTGTGCTGCA TGACGGTG                                                     18
```